(19) Europäisches Patentamt
European Patent Office
Office européen des brevets

(11) Publication number : **0 285 655 B1**

# (12) EUROPEAN PATENT SPECIFICATION

(45) Date of publication of patent specification : **15.04.92 Bulletin 92/16**

(51) Int. Cl.$^5$ : **C07D 215/56**

(21) Application number : **87907128.0**

(22) Date of filing : **14.10.87**

(86) International application number : **PCT/HU87/00044**

(87) International publication number : **WO 88/02748 21.04.88 Gazette 88/09**

## (54) PROCESS FOR THE PREPARATION OF QUINOLINE-CARBOXYLIC ACID DERIVATIVES.

(30) Priority : **15.10.86 HU 429286**
**01.10.87 HU 429286**

(43) Date of publication of application : **12.10.88 Bulletin 88/41**

(45) Publication of the grant of the patent : **15.04.92 Bulletin 92/16**

(84) Designated Contracting States : **AT CH FR IT LI NL SE**

(56) References cited :
**US-A- 4 559 341**
**CHEMICAL ABSTRACTS, vol. 79, no. 15, October 15, 1973, Columbus, Ohio, USA; M.A. KRAUS: "Formylation of aliphatic amines by dimethylformamide", p. 383, column 2, abstract-no. 91 540w**
**CHEMICAL ABSTRACTS, vol. 104, no. 11, March 17, 1986, Columbus, Ohio, USA; R. SAITO et al.: "Recovery of piperazine", p. 666, column 1, abstract-no. 88 605a**

(73) Proprietor : **CHINOIN Gyogyszer és Vegyészeti Termékek Gyára RT.**
**To utca 1-5**
**H-1045 Budapest IV (HU)**

(72) Inventor : **FRANK, Judit**
**Szolo köz 2**
**H-1032 Budapest (HU)**
Inventor : **BERES, Jozsef Károlyné**
**Baba utca 7**
**H-1025 Budapest (HU)**
Inventor : **KULCSAR, Gábor**
**Szlovák utca 100**
**H-1162 Budapest (HU)**

(74) Representative : **Patentanwälte Beetz sen. - Beetz jun. Timpe - Siegfried - Schmitt-Fumian-Mayr**
**Steinsdorfstrasse 10**
**W-8000 München 22 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The invention relates to a new and simple process for the preparation of quinoline carboxylic acid derivatives as well as hydrates and salts, particulary pharmaceutically acceptable salts thereof, the carboxylic acids having the general formula I,

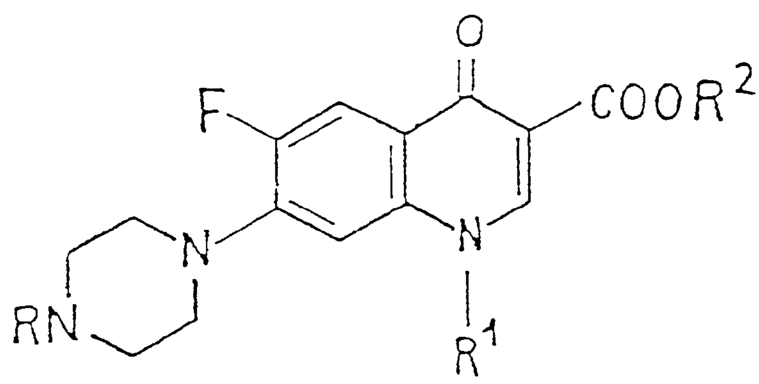

(I)

wherein R is hydrogen or formyl,

$R^1$ represents hydrogen or a straight chain or cyclic alkyl group having 1 to 4 carbon atoms, which may be substituted by a halogen atom, a hydroxy group or an amino group; or a $CH_3$-NH group,

and

$R^2$ is hydrogen or an alkyl group having 1 to 4 carbon atoms,

from compounds of the general formula II,

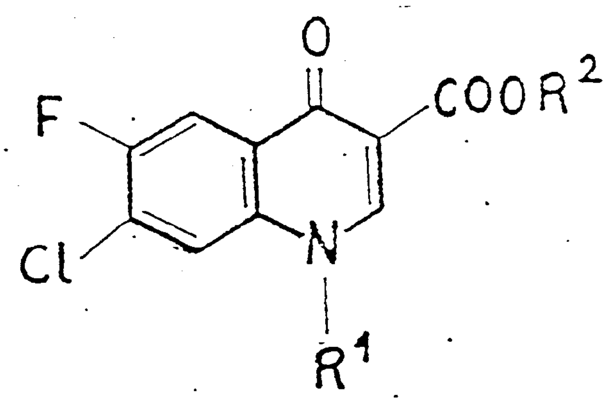

(II),

wherein $R^1$ and $R^2$ are as defined above; this process is characterized by

(A) reaction of the compound of the general formula II or an acid addition salt thereof with piperazine in dimethylformamide to obtain a compound of the general formula III,

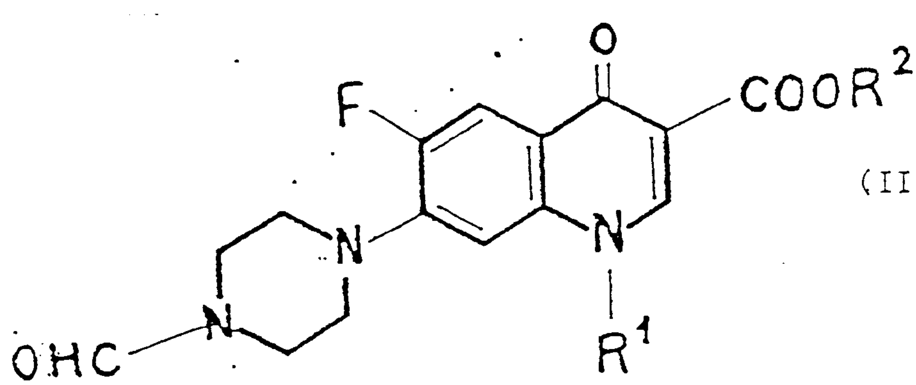

(III),

wherein $R^1$ and $R^2$ are as defined above, and optionally

(B) acidic or alkaline treatment of the compound of formula III.

The preparation of compounds of the general formula III was carried out up to the present by reacting a quinoline carboxylic acid derivative of the general formula II with N-formylpiperazine in dimethyl sulfoxide as solvent (BE-A-870576). Further a process is known to form the formyl-free derivative by formylation with formic acid in a yield of 50 % (J. Med. Chem. 23 (1980) 1358). This latter process is very corrosive because of the application of formic acid.

The disadvantages of these prior art methods reside in that they are carried out in several steps and in expensive solvents such as pyridine, dimethyl sulfoxide, etc., and the yields are very low.

It is the object of the present invention to provide a process for the preparation of the compounds of formula I.

This object is achieved as defined above according to claim 1. The dependent claims relate to advantageous embodiments.

Among the compounds of formula I, 1-ethyl-6-fluoro-7-(4-formyl)-piperazinyl-1,4-dihydro-4-oxoquinoline-3-carboxylic acid possesses a very significant antibacterial activity. It is effective against Pasteurella multocida in a minimal inhibitory concentraction of 0.25 μg/ml, against various Bacillus strains (e.g. B. subtilis, B. licheniformis) in a minimal inhibitory concentration of 0.5-0.75 μg/ml, and similarly in a minimal inhibitory concentration of 0.75 μg/ml against Shibella sonnei and Salmonella cholerae-suis strains.

According to the present invention, the formylation can be carried out advantageously with heating.

Higher temperatures (120-153 °C) result in a shorter reaction time. The yield of the reaction can be increased by using an acidic medium. The acidic medium can be achieved by using an acid addition salt of the compound of the general formula II or advantageously by carrying out the reaction in the presence of an acid, advantageously in the presence of hydrochloric or sulfuric acid.

The processing of the reaction mixture and the recovery of the product can be effected in a very simple way. The dimethylformamide applied as solvent and as a reactant is removed under reduced pressure, and it may be used repeatedly. To the residue obtained water is added, and the precipitate is recovered by filtration.

Among the compounds of the general formula I, those of the general formula IV

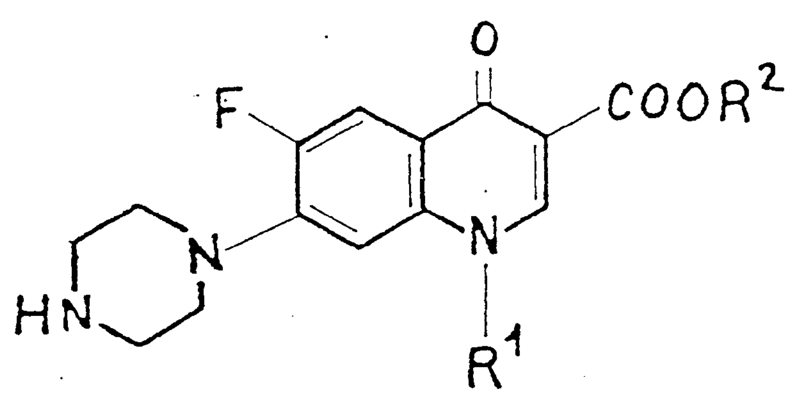

(IV)

possess valuable antibacterial activity (cf. e.g. Antimicrob. Agents Chemother. 1985, 581-586). They can be prepared from compounds of the general formula III containing the formyl group by acidic or alkaline treatment, advantageously with hydrazine, preferably with hydrazine-hydrate, optionally in the presence of an acid (e.g. acetic acid).

As acids, mineral acids, e.g. hydrochloric acid, sulfuric acid, phosphoric acid, etc., are applied in a suitable dilution. As alkali the hydroxides and carbonates of the alkali and alkaline earth metals are used. The removal of the formyl group is carried out in an organic solvent and/or in water, advantageously in an alcohol-water mixture. The most advantageous solvent is a 3:1 mixture of isopropanol and water. The reaction is carried out at a temperature of 25-100 °C, advantageously at the boiling point; higher temperatures result in a shorter reaction time.

The product can be obtained advantageously after neutralization of the acid or alkali excess of the reaction mixture by filtration. If desired the product can be purified by recrystallization. The compounds of the general formula I can be obtained in the form of hydrates, or the hydrates can be formed by setting free from their salts or therapeutically acceptable salts can be formed.

An important aspect of the present process is the fact that the formyl group can be removed from the compounds of the general formula III by reacting with hydrazine. This corresponds to a new type process, which

leads to a more uniform and pure product as compared to the hydrolysis processes of the prior art.

Further details of the present process are shown in the examples.

**Example 1**

5.4 g (0.02 mol) of 1-ethyl-6-fluoro-7-chloro-1.4-dihydro-4-oxoquinoline-3-carboxylic acid and 10.4 g (0.12 mol) of piperazine in 120 ml of dimethylformamide are heated for 6 h at 145 °C. Thereafter the solvent is removed under reduced pressure, and 300 ml of water are added to the residue. The precipitate is obtained by filtration. Thus 4.6 g of 1-ethyl-6-fluoro-7-(4-formyl)-piperazinyl-1,4-dihydro-4-oxoquinoline-3-carboxylic acid are obtained; melting point 270 °C. After recrystallisation from dimethylformamide the melting point raises to 285-286 °C.

**Example 2**

1.0 g (0.0029 mol) of 1-ethyl-6-fluoro-7-(4-formyl)piperazinyl-1,4-dihydro-4-oxoquinoline-3-carboxylic acid is suspended in 40 ml of a 3:1 mixture of isopropanol and water, and 10 ml of 1 N hydrochloric acid are added. The reaction mixture is boiled for 5 h. After cooling, the product crystallizes in the form of a hydrochloric acid salt and can be removed by filtration, or after neutralization with 1 N sodium hydroxide solution the precipitated product is filtered. After drying 0.58 g of 1-ethyl-6-fluoro-7-piperazinyl-1,4-dihydro-4-oxoquinoline-3-carboxylic acid are obtained; melting point 222 °C.

**Example 3**

1.0 g (0.0029 mol) of 1-ethyl-6-fluoro-7-(4-formyl)piperazinyl-1,4-dihydro-4-oxoquinoline-3-carboxylic acid is suspended in 40 ml of a 3:1 mixture of isopropanol and water, and 5 ml of aqueous 1 N sodium hydroxide solution are added. The reaction mixture is boiled for 8 h. After cooling the solution is neutralized with a diluted hydrochloric acid solution. The precipitated product is filtered. Thus, 0.6 g of 1-ethyl-6-fluoro-7-piperazinyl-1,4-dihydro-4-oxoquinoline-3-carboxylic acid are obtained; melting point 218 °C.

**Example 4**

1.0 g (0.0029 mol) of 1-ethyl-6-fluoro-7-(4-formyl)piperazinyl-1,4-dihydro-4-oxoquinoline-3-carboxylic acid is suspended in 50 ml of a 3:1 mixture of isopropanol and water, and 0.5 ml (0.010 mol) of 98 % hydrazine-hydrate and 0.6 ml (0.010 mol) of acetic acid are added. The reaction mixture is heated at 100 °C for 6 h. The product crystallizing from the cooled solution is obtained by filtration. Thus 0.827 g of 1-ethyl-6-fluoro-7-piperazinyl-1,4-dihydro-4-oxoquinoline-3-carboxylic acid are obtained; melting point 222 °C.

**Example 5**

1.35 g (0.005 mol) of 1-ethyl-6-fluoro-7-chloro-1,4-dihydro-4-oxoquinoline-3-carboxylic acid, 2.6 g (0.03 mol) of piperazine and 1 ml of concentrated hydrochloric acid are heated at boiling temperature in 30 ml of dimethylformamide for 4 h. The solvent is then removed under reduced pressure, and 80 ml of water are added to the residue. The precipitated product is filtered and washed with water. After drying 1.42 g (82 %) of 1-ethyl-6-fluoro-7-(4-formyl)-piperazinyl-1,4-dihydro-4-oxoquinoline-3-carboxylic acid are obtained; melting point 288 °C.

## Claims

1. A process for the preparation of quinoline carboxylic acids of the general formula I and their hydrates and salts,

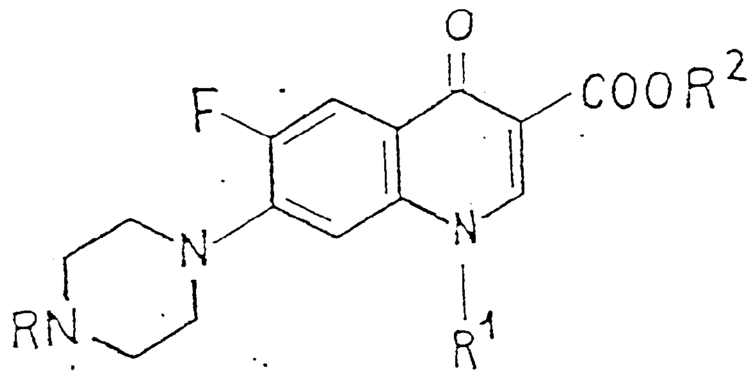

(I),

wherein R is hydrogen or formyl,

$R^1$ represents hydrogen or a straight chain or cyclic alkyl group having 1 to 4 carbon atoms, which may be substituted by a halogen atom, a hydroxy group or an amino group; or a $CH_3$-NH group,

and

$R^2$ is hydrogen or an alkyl group having 1 to 4 carbon atoms, from compounds of the general formula II,

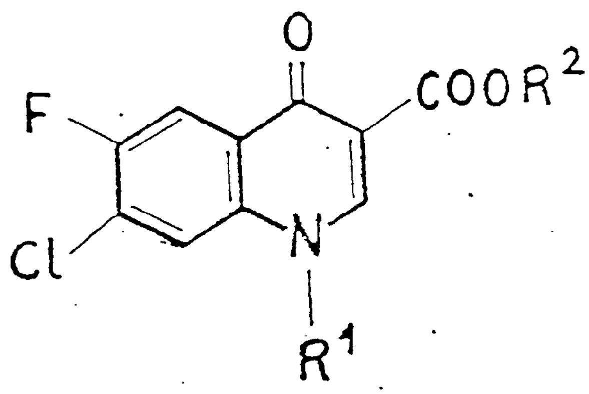

(II),

wherein $R^1$ and $R^2$ are as in formula I,

**characterized** by

(A) reaction of the compound of the general formula II or an acid addition salt thereof with piperazine in dimethylformamide to obtain a compound of the general formula III,

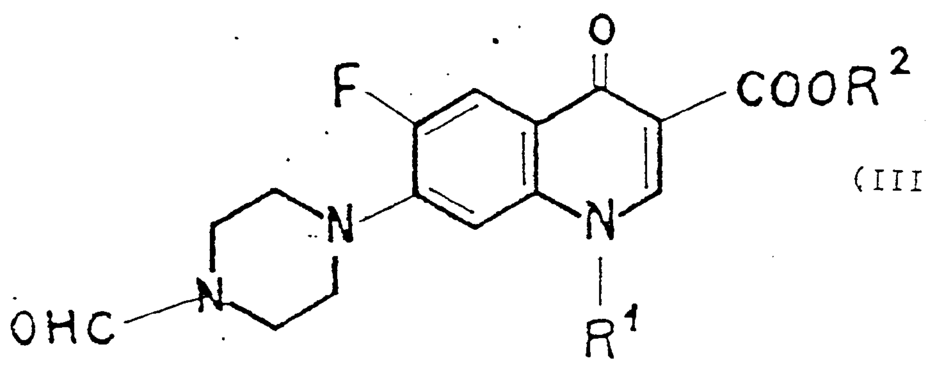

(III),

wherein $R^1$ and $R^2$ are as defined above, and optionally

(B) acidic or alkaline treatment of the compound of formula III.

2. The method according to claim 1, characterized in that step A is carried out in an acidic medium.

3. The method according to claim 2, characterized in that hydrochloric acid or sulfuric acid are used for the acidic medium in step A.

4. The method according to one of claims 1 to 3, characterized in that a mineral acid is used for the acidic treatment in step B.

5. The method according to one of claims 1 to 3, characterized in that an alkali is used for the alkaline treatment in step B.

6. The method according to one of claims 1 to 3, characterized in that hydrazine is used for the alkaline treatment in step B.

7. The method according to one of claims 1 to 6, characterized in that step B is carried out in an isopropanol-water mixture.

**Patentansprüche**

1. Verfahren zur Herstellung von Chinolincarbonsäuren der allgemeinen Formel I und ihren Hydraten und Salzen,

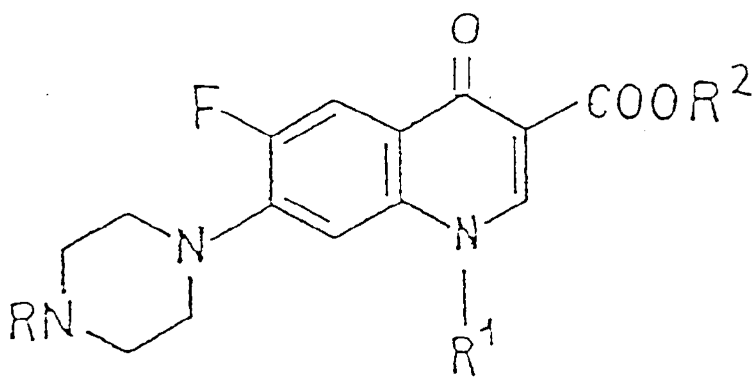

(I),

worin bedeuten:

R Wasserstoff oder Formyl,

$R^1$ Wasserstoff oder eine geradkettige oder cyclische Alkylgruppe mit 1 bis 4 Kohlenstoffatomen, die mit einem Halogenatom, einer Hydroxygruppe oder einer Aminogruppe substituiert sein kann; oder eine $CH_3$-NH-Gruppe,

und

$R^2$ Wasserstoff oder eine Alkylgruppe mit 1 bis 4 Kohlenstoffatomen, aus Verbindungen der allgemeinen Formel II,

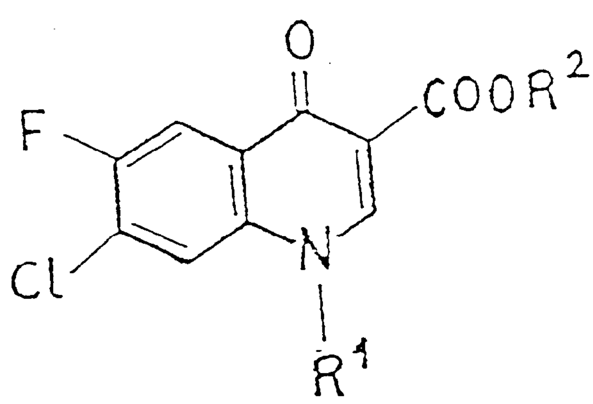

(II),

worin $R^1$ und $R^2$ die gleiche Bedeutung wie in Formel I besitzen,
**gekennzeichnet** durch

(A) Umsetzung der Verbindung der allgemeinen Formel II oder eines ihrer Säureadditionssalze mit Piperazin in Dimethylformamid unter Erhalt einer Verbindung der allgemeinen Formel III,

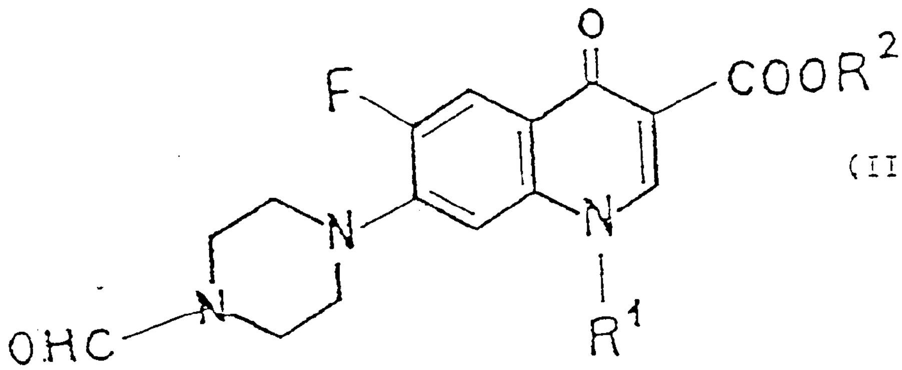

(III),

worin $R^1$ und $R^2$ die gleiche Bedeutung wie oben besitzen, und gewünschtenfalls
(B) saure oder alkalische Behandlung der Verbindung der Formel III.

2. Verfahren nach Anspruch 1, dadurch gekennzeichnet, daß Stufe A in einem sauren Medium durchgeführt wird.

3. Verfahren nach Anspruch 2, dadurch gekennzeichnet, daß Salzsäure oder Schwefelsäure für das saure Medium in Stufe A verwendet werden.

4. Verfahren nach einem der Ansprüche 1 bis 3, dadurch gekennzeichnet, daß eine Mineralsäure für die saure Behandlung in Stufe B verwendet wird.

5. Verfahren nach einem der Ansprüche 1 bis 3, dadurch gekennzeichnet, daß für die alkalische Behandlung in Stufe B eine Alkaliverbindung verwendet wird.

6. Verfahren nach einem der Ansprüche 1 bis 3, dadurch gekennzeichnet, daß für die alkalische Behandlung in Stufe B Hydrazin verwendet wird.

7. Verfahren nach einem der Ansprüche 1 bis 6, dadurch gekennzeichnet, daß Stufe B in einem Isopropanol-Wasser-Gemisch durchgeführt wird.

## Revendications

1. Un procédé pour la préparation d'acides quinoléine carboxylique de formule générale I et de leurs hydrates et sels,

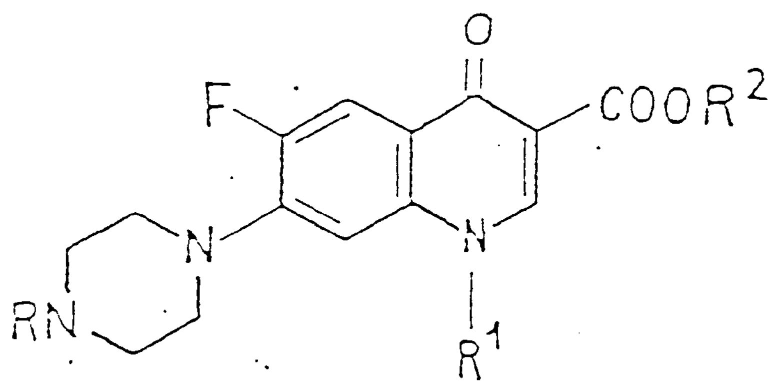

(I),

dans laquelle

R est l'hydrogène ou un groupement formyle,

$R^1$ représente l'hydrogène ou un groupement alkyle à chaîne linéaire ou cyclique ayant de 1 à 4 atomes de carbone, lesquels peuvent être substitués par un atome halogène, un groupement hydroxy ou un groupement amino; ou un groupement $CH_3$-NH,

et

$R^2$ est l'hydrogène ou un groupement alkyle ayant de 1 à 4 atomes de carbone à partir de composés de formule générale II,

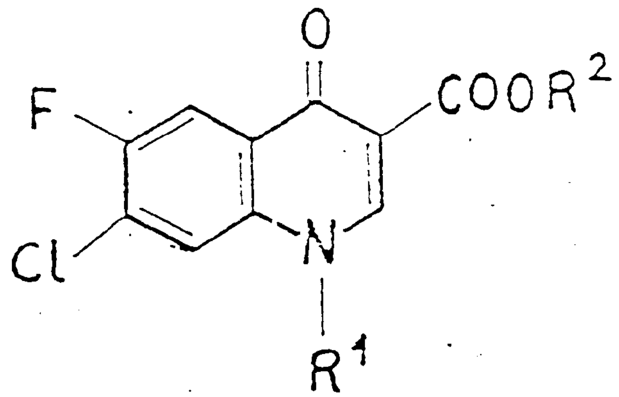

(II)

dans laquelle $R^1$ et $R^2$ sont comme définis plus haut;
caractérisé par

(A) la réaction d'un composé de formule générale II ou d'un sel d'addition d'acide de celui-ci avec une pipérazine dans du diméthylformamide pour obtenir un composé de formule générale III,

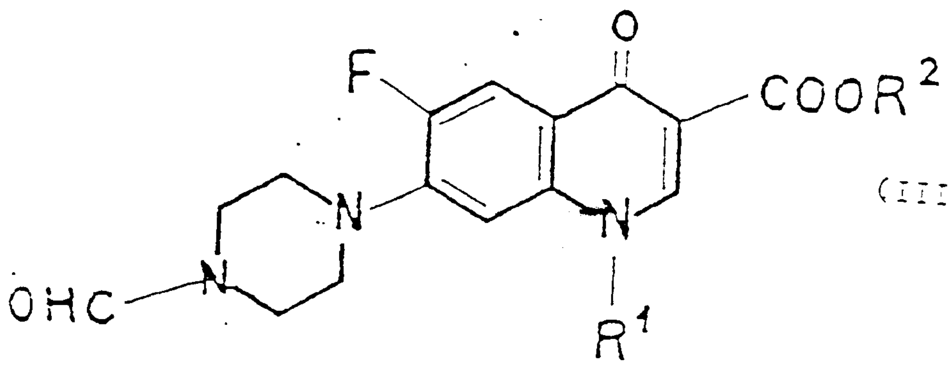

(III)

dans laquelle $R^1$ et $R^2$ sont comme définis ci-dessus, et facultativement

(B) un traitement acide ou alcalin du composé de formule III.

2. La méthode selon la revendication 1, caractérisée en ce que l'étape A est conduite dans un milieu acide

3. La méthode selon la revendication 2, caractérisée en ce qu'on utilise de l'acide chlorhydrique ou sulfurique pour le milieu acide de l'étape A.

4. La méthode selon l'une des revendications 1 à 3, caractérisée en ce qu'on utilise un acide minéral pour le traitement acide de l'étape B.

5. La méthode selon l'une des revendications 1 à 3, caractérisée en ce qu'on utilise une base pour le traitement alcalin de l'étape B.

6. La méthode selon l'une des revendications 1 à 3, caractérisée en ce qu'on utilise de l'hydrazine pour le traitement alcalin de l'étape B.

7. La méthode selon l'une des revendications 1 à 6, caractérisée en ce que l'étape B est conduite dans un mélange isopropanol-eau.